# EUROPEAN PATENT SPECIFICATION

(11) **EP 2 290 369 B1**
(45) Date of publication and mention of the grant of the patent: **14.08.2013**
(21) Application number: 10181451.5
(22) Date of filing: 21.12.2005
(51) Int. Cl.: G01N 33/68

(54) **Methods for proteomic profiling using non-natural amino acids**
Verfahren zur proteomischen Profilierung mithilfe nichtnatürlicher Aminosäuren
Méthodes de profilage protéomique utilisant des acides aminés non naturels

(30) Priority: 22.12.2004 US 638856 P; 05.04.2005 US 668471 P
(43) Date of publication of application: 02.03.2011
(62) Divisional of application: 05855111.0
(73) Proprietor: CALIFORNIA INSTITUTE OF TECHNOLOGY, Pasadena, California 91115 (US)
(72) Inventor: Link, Aaron J., Princeton, NJ 08544 (US); Schuman, Erin, Pasadena, CA 91106 (US); Tirrell, David A., Pasadena, CA 91106 (US); Dieterich, Daniela C., 39118 Magdeburg (DE)
(74) Representative: Couchman, Jonathan Hugh

(56) References cited:
- KIICK KRISTI L ET AL: "Incorporation of azides into recombinant proteins for chemoselective modification by the Staudinger ligation.", PROCEEDINGS OF THE NATIONAL ACADEMY OF SCIENCES OF THE UNITED STATES OF AMERICA. 8 JAN 2002, vol. 99, no. 1, 8 January 2002 (2002-01-08), pages 19-24, XP002409869, ISSN: 0027-8424
- LINK A JAMES ET AL: "Presentation and detection of azide functionality in bacterial cell surface proteins.", JOURNAL OF THE AMERICAN CHEMICAL SOCIETY. 1 SEP 2004, vol. 126, no. 34, 1 September 2004 (2004-09-01), pages 10598-10602, XP002409870, ISSN: 0002-7863
- LINK A JAMES ET AL: "Cell surface labeling of Escherichia coli via copper(I)-catalyzed [3+2] cycloaddition.", JOURNAL OF THE AMERICAN CHEMICAL SOCIETY. 17 SEP 2003, vol. 125, no. 37, 17 September 2003 (2003-09-17), pages 11164-11165, XP002409871, ISSN: 0002-7863
- OLSEN JESPER V ET AL: "HysTag--a novel proteomic quantification tool applied to differential display analysis of membrane proteins from distinct areas of mouse brain.", MOLECULAR & CELLULAR PROTEOMICS : MCP. JAN 2004, vol. 3, no. 1, January 2004 (2004-01), pages 82-92, XP002409872, ISSN: 1535-9476
- GYGI S P ET AL: "Quantitative analysis of complex protein mixtures using isotope-coded affinity tags.", NATURE BIOTECHNOLOGY. OCT 1999, vol. 17, no. 10, October 1999 (1999-10), pages 994-999, XP001010578, ISSN: 1087-0156

### SEQUENCE LISTING

<110> California Institute of Technology Dieterich, Daniela C. Tirrell, David A. Schuman, Erin Link, Aaron J.
<120> METHODS FOR PROTEOMIC PROFILING USING
   NON-NATURAL AMINO ACIDS
<130> CTCH-PWO-034
<140> PCT/US05/046496
   <141> 2005-12-21
<150> US 60/638,856
   <151> 2004-12-22
<150> US 60/668,471
   <151> 2005-04-05
<160> 2
<170> FastSEQ for windows version 4.0
<210> 1
   <211> 8
   <212> PRT
   <213> Artificial Sequence
<220>
   <223> FLAG epitope tag
<400> 1
   Asp Tyr Lys Asp Asp Asp Asp Lys
1 5
<210> 2
   <211> 11
   <212> PRT
   <213> Artificial sequence
<220>
   <223> FLAG epitope tag with a linker sequence
<400> 2
   Gly Gly Ala Asp Tyr Lys Asp Asp Asp Asp Lys
    1               5                   10

## Description

### Background of the Invention

Cells respond to fluctuations in their environment by changing the set of proteins they express. Understanding such changes is important for the understanding of cellular processes and for the understanding of how pharmaceuticals alter such expression patterns. Alterations in protein synthesis and degradation enable cells (such as neurons), to adapt to changing external conditions.

As a specific example, in neurons, there is increasing evidence that local dendritic protein synthesis is used to allow individual synapses to respond dynamically to the environmental changes that accompany the establishment, maintenance and plasticity of synaptic connections. It is now well established that the essential components of the translational machinery as well as mRNAs are present at or near synapses. There is also now ample evidence that dendrites can synthesize new proteins. Nevertheless, only a handful of dendritically translated proteins have been identified thus far, largely through candidate-based approaches inspired by a particular laboratory's interest in a specific protein. The current piecemeal accrual of knowledge about the dendritically synthesized proteome has hindered the understanding of the logic of local translation.

Most of the current understanding of the dendritic proteome comes not from protein data, but rather from catalogs of dendritic mRNAs. Yet, the dendritic mRNA population has been explored via either candidate based approaches (does the mRNA for protein "x" localize to dendrites) or mRNA generation from single neuritic process PCR experiments. A major shortcoming of these data is that this approach only provides a rather short list of transcripts, not an exhaustive list of proteins. A focus on translated proteins is essential for an accurate picture of how the synapse and the dendrite can mount a response to local changes in the environment.

This problem is far from being neuron-specific. In fact, there is a general need to effectively determine the protein expression profiles, of especially newly synthesized proteins, in two samples, such as diseased sample vs. normal sample, a control sample and a drug treated sample, the same sample observed at two different time points, *etc.*

Proteomic approaches, including differential 2D-gel electrophoresis (Freeman & Hemby, Proteomics for protein expression profiling in neuroscience. Neurochem Res 29: 1065-1081, 2004; Lilley & Friedman, All about DIGE: quantification technology for differential-display 2D-gel proteomics. Expert Rev Proteomics 1: 401-409, 2004) (DIGE), isotope-coded affinity tags (ICAT) (Gygi et al., Quantitative analysis of complex protein mixtures using isotope-coded affinity tags. Nat Biotechnol 17: 994-999, 1999), quantitative proteomic analysis using samples from cells grown in ¹⁴N or ¹⁵N-media (Washburn et al., Analysis of quantitative proteomic data generated via multidimensional protein identification technology. Anal Chem 74: 1650-1657, 2002) and stable isotope labeling by amino acids in cell culture (Andersen et al., Nucleolar proteome dynamics. Nature 433: 77-83, 2005; Ong et al., Stable isotope labeling by amino acids in cell culture, SILAC, as a simple and accurate approach to expression proteomics. Mol Cell Proteomics 1: 376-386, 2002) (SILAC), have been developed to compare the protein expression profiles of cells in different states. In addition, approaches focusing on posttranslational modifications (Chang et al., Analysis of protein phosphorylation by hypothesis-driven multiple-stage mass spectrometry. Anal Chem 76: 4472-4483, 2004; Garcia et al., Analysis of protein phosphorylation by mass spectrometry. Methods 35: 256-264, 2005; Peters et al., Exploring the phosphoproteome with mass spectrometry. Mini Rev Med Chem 4: 313-324, 2004) have emerged to explore the "phosphoproteome" and "glycoproteome" of a cell.

There are, however, drawbacks associated with approaches like DIGE, ICAT and SILAC. For example, 2D-gel electrophoresis has a limited ability to detect proteins of low abundance, membrane proteins, or proteins with unusual isoelectric points or molecular weights. With ICAT, valuable protein information including post-translational modifications can be lost, as only cysteine-containing peptides are analyzed. SILAC critically depends on the ideally complete incorporation of a given "light" or "heavy" form of an essential amino acid into two cell pools, a process that is expensive, time-consuming and restricted to cell lines which can be kept in an artificial medium. Common to all these approaches is a prerequisite for decreasing the sample complexity before mass spectrometrical analysis. This is accomplished by cutting out single bands or spots (in the case of DIGE and SILAC) or by the affinity-purification of biotinylated cysteine-containing peptides (ICAT). Most importantly, however, these approaches lack the ability to enrich for newly synthesized proteins. Enrichment for newly synthesized proteins has at least two advantages: (i) it permits an analysis of the primary protein synthesis response to internal and external cues / stimuli, and, (ii) it decreases the complexity of the sample, potentially enabling the identification of proteins expressed at low levels. In order to understand the primary response of cells to internal and external cues the ability to differentiate the newly synthesized proteome from the pre-existing protein pool is desirable. Currently available approaches using radioactively labeled amino acids can detect newly synthesized proteins but do not enrich for them. Furthermore, it is problematic to directly use radiolabeled samples for mass spectral analyses due to radioactive contamination issues.

Finally, methods focusing specifically on mRNA expression profiles, only indirectly reflect the protein population in a sample, since not all mRNAs in the sample are necessarily translated at any given condition. Even for translated mRNAs, the degree of translation is often not predicted by the transcriptional response.

Accordingly, there is a need for new methods, systems, and reagents for analyzing (new) protein expression.

### Summary of the Invention

A first aspect of the invention provides a functional reagent comprising: (1) a reactive group for reacting with a non-natural amino acid incorporated into a protein; (2) an affinity moiety or a fluorescent moiety; and, (3) one or more cleavable groups situated between the reactive group and the affinity moiety or the fluorescent moiety; wherein the reactive group is one of the following:
a) an azido group; or
b) a ketone or an aldehyde moiety; and
c) a terminal alkyne moiety and wherein the one or more cleavable groups are selected from: photo-cleavable groups; groups which have a recognition sequence for a protease; groups which have a recognition sequence for an endonuclease; and chemically cleavable groups selected from diols, disulfides, diazo groups, esters, sulfones, and groups comprising methionine.

In one embodiment, the functional reagent is sufficiently membrane permeable to diffuse into a live cell.

In one embodiment, the functional reagent is substantially non-toxic.

In one embodiment, the reactive group reacts with a non-natural amino acid incorporated in a protein.

In one embodiment, the functional reagent further comprises an antigenic moiety that can be recognized by an antibody. In one embodiment, the antigenic moiety is an epitope tag, such as a FLAG tag, an HA tag, a His6 tag, etc.

In one embodiment, the FLAG tag comprises one or more cleavage sites for a sequence-specific protease, such as trypsin.

In one embodiment, the FLAG tag is situated between the affinity moiety and the second reactive group.

In one embodiment, the reactive group and the affinity moiety are linked by one or more cleavable functional groups, such as photo-cleavable groups, chemically cleavable groups, or enzymatically cleavable groups.

In one embodiment, the reactive group and the affinity moiety are linked by one or more cleavage sites for a sequence-specific protease, such as Factor Xa or PreScission Protease.

In one embodiment, the affinity moiety is biotin.

In one embodiment the affinity moiety is biotin, wherein the FLAG tag comprises two cleavage sites for a sequence-specific protease, and wherein the FLAG tag is situated between the affinity moiety and the reactive group.

In one embodiment, the linker is a photo-cleavable linker.

Another aspect of the invention provides the use of a reactive group of a functional reagent to react with a non-natural amino acid incorporated into a protein, wherein the functional reagent comprises:
(1) the reactive group; and
(2) an affinity moiety or a fluorescent moiety;
   wherein the reactive group is one of the following:
   (a) an azido group;
   (b) a ketone or an aldehyde moiety; or
   (c) a terminal alkyne moiety.

The embodiments described above, including those described under different aspects of the invention, are contemplated to be applicable for all aspects of the inventions wherever appropriate.

### Brief Description of the Figures

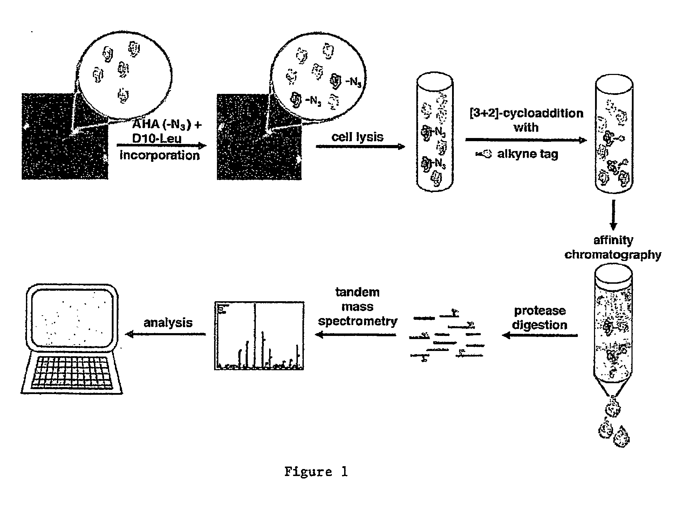

Figure 1 : Overview of the protein identification procedure using non-natural amino acids, such as azidohomoalanine (AHA). Cells are challenged in the presence of AHA to allow for protein synthesis with AHA-incorporation ("+/-" represents the presence / absence of a modulator of activity). After incubation, cells are lysed or undergo a subcellular fractionation for biochemical enrichment of specific cellular compartments followed by lysis. Lysates are then coupled to an alkyne-bearing affinity tag, followed by affinity chromatography to enrich for AHA-incorporated proteins. Purified proteins are digested with a protease, and the resulting peptides are analyzed by tandem mass spectrometry to obtain experimental spectra. Different search programs are used to match the acquired spectra to protein sequences.

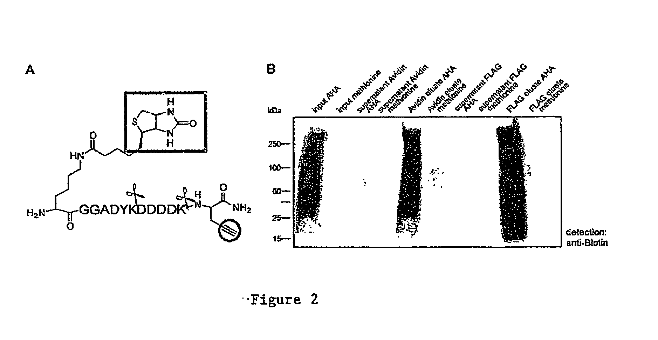

Figure 2: Generation of a tandem featured alkyne tag for two-step purification of AHA incorporated proteins. Structure of a Biotin-FLAG-alkyne affinity reagent. Biotin (square), alkyne (square circle) as well as the tryptic cleavage sites (scissors) are indicated. The FLAG epitope DYKDDDDK (SEQ ID NO: 1) is separated from the biotin moiety by a short linker (GGA). (B) Western blot analysis for tandem purified biotinylated proteins using the Biotin-FLAG-alkyne tag. Cell lysates from both AHA and methionine incubated HEK293 cells were subjected to [3+2]-cycloaddition with the Biotin-FLAG-alkyne tag and subsequently purified using monomeric avidin and anti-FLAG-M2 affinity matrices. SDS was used in both steps to elute the proteins from the resin. SDS was removed from avidin-eluates by ultrafiltration and neutralization with Triton X-100 before incubation with the FLAG-M2 affinity matrix. Sizes of marker proteins are indicated on the left margin.

### Detailed Description of the Invention

### 1. Overview

The invention provides reagents for labeling, detecting / monitoring, isolating, and/or identifying the translation products in a mammalian cell.

New proteins synthesized in a cell can be labeled by contacting the cell with a non-natural amino acid that can be incorporated into proteins in the cell in place of a naturally occurring amino acid. The non-natural amino acid generally has a first reactive moiety that can be used to label proteins that incorporate such an amino acid residue. Generally, the first reactive moiety has a functional group that can be specifically reacted with a second reactive moiety on a reagent, which reagent further comprises a functional moiety, such as a fluorescent moiety, an affinity moiety, *etc*. Once the first reactive moiety on the non-natural amino acid reacts with the second reactive moiety on the reagent, the functional group becomes associated with the protein containing the non-natural amino acid, thereby facilitating the labeling, detecting / monitoring, isolating, and/or identifying the translation products in a cell or tissue.

Preferably, the first and the second reactive moieties react with each other with relatively high specificity, such that the reaction may occur without undesirable side-reactions or interferences due to the presence of other proteins without the non-natural amino acids (*e.g*., reactions between the reagent and the unlabeled proteins).

The cells may be cultured *in vitro,* and the non-natural amino acid and the reagent are provided directly to the cell culture medium. The cells may even be lysed or partially permeablized to facilitate the accessibility of the non-natural amino acid and the reagent to the translation machinery. Alternatively, the cells may be cells of a tissue or a live organism, wherein the non-natural amino acid and the reagent are administered to the organism (*e.g*., by feeding, injection, etc.).

The non-natural amino acid may be incorporated into proteins by the endogenous translation machinery of cells or tissue, including the endogenous amino-acyl tRNA synthetases (AARS) that can take the non-natural amino acid as a substrate, and charge it to an endogenous tRNA; and the endogenous ribosome. Preferably, the AARS and/or the tRNA are for a natural amino acid to which the non-natural amino acid is a structural homolog.

Alternatively, the non-natural amino acid is incorporated into proteins of the cells / tissue with at least partial aid from non-endogenous translation machinery. For example, the non-natural amino acid may be charged to a tRNA (endogenous, modified endogenous, or tRNA from a different species, *etc*.) by a non-endogenous AARS, which AARS may be engineered specifically to take the non-natural amino acid as a substrate. See, for example, US 2004-0053390 A1, US 2004-0058415 A1, WO 03/073238 A2, U.S. Pat. No. 6,586,207 (all incorporated herein by reference). The charged non-natural amino acid - tRNA may be produced *in vitro* and provided to the cell or tissue. Alternatively, the modified AARS and/or tRNA may be provided to the cells / tissue, for example, by introducing into the cells a polynucleotide encoding the AARS and/or tRNA, to charge the non-natural amino acid *in vivo.*

The non-natural amino acid preferably contains one or more of the following desirable characteristics: (1) relatively permeable through bio-membranes, such as plasma membranes, such that it can be directly provided in tissue culture medium or administered to a live organism for direct uptake by the cell; (2) relatively stable *in vitro* and *in vivo*; (3) being a structural homolog of one or more natural amino acids, such as methionine or phenylalanine (In certain embodiments, the natural amino acid is an essential amino acid, so that the cells can be made auxotrophic for that natural amino acid; in certain embodiments, the natural amino acid is relatively rarely used in proteins); (4) capable of being charged directly to an endogenous tRNA by an endogenous AARS; (5) the charged non-natural amino acid - tRNA complex can be readily incorporated into proteins by endogenous ribosomes; (6) the structure of the non-natural amino acid is such that, upon incorporation into the proteins, it does not substantially affect the folding and/or biological function of any proteins incorporating the non-natural amino acid; and (7) being substantially non-toxic and biologically inert.

The first reactive moiety on the non-natural amino acid preferably can react with the second reactive moiety of another reagent under relatively mild conditions, such as physiological conditions with a relatively neutral / physiological pH and temperature. One or more catalysts (preferably non-toxic) may also be provided to facilitate the reaction under such conditions.

However, the reaction may occur in more harsh conditions, with any type of suitable catalyst, since proteins labeled by the non-natural amino acid may be isolated or be present in a cell lysate or an *in vitro* translation system before the reaction between the first and second reactive moieties occur.

Cells / tissues incorporated with non-natural amino acids may be fixed or permeated after the incorporation step, before the agent with the second reactive group and functional moieties are provided (*e.g*., in the case of immunostairung, *etc*.). Since the reaction between the first and the second reactive groups occur only after the fixation of cells / tissues, there are fewer limitations regarding the type of second reactive groups and/or catalysts that may be used in the methods of the invention.

The methods of the disclosure can be used to incorporate non-natural amino acids (e.g., those with azide moiety, such as AHA, or those with terminal alkyne groups, *etc*.) into proteins / polypeptides translated *in vitro.* Either endogenous or ortholog AARS / tRNA pairs may be used to charge the non-natural amino acids. Since there is no issue of membrane permeability, and much less of an issue of toxicity (if any at all), the range of non-natural amino acids and the types of catalysts that can be used in these embodiments are wider than those for intact cell / tissue use.

The amino acid used may be azidohomoalanine (AHA) or an analog thereof. AHA is an analog of the essential natural amino acid methionine, which is relatively rarely used in proteins. Other methionine / AHA homologs that can be used in the invention include: azidoalanine, azidonorvaline, and azidonorleucine, *etc*. See Link et al., J. Am, Chem. Soc. 126: 10598-10602, 2004 (incorporated herein by reference).

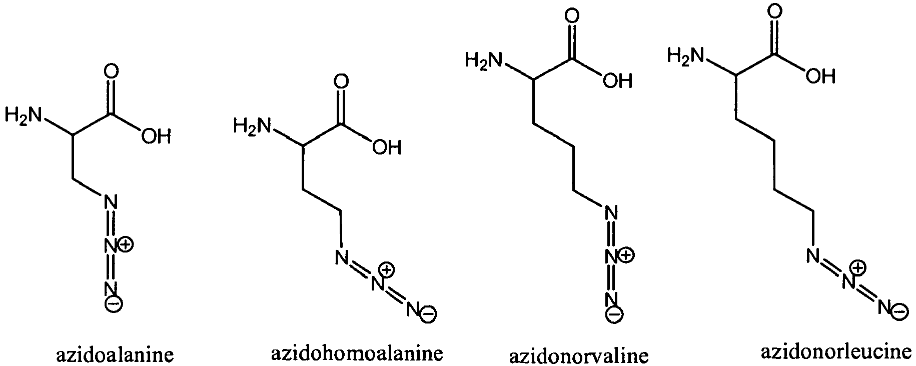

The non-natural amino acid may have a ketone moiety. The non-natural amino acid may have a diboronic acid moiety.

The concentration of the non-natural amino acid used to contact cells in culture (or the amount administered to an animal) can be selected to optimize the detection of newly synthesized proteins.

Once the non-natural amino acid is incorporated into the proteins of the cells, proteins from the cells are then treated with a reagent that can become associated with the non-natural amino acid. The reagent comprises a second reactive moiety that can react with the first reactive moiety of the non-natural amino acid. For example, when the non-natural amino acid has an azido moiety, the reagent will generally have an alkyne moiety, and vice versa

In certain embodiments, the reagent is an affinity reagent that also comprises at least one affinity moiety, such as biotin, that can be used to isolate the non-natural amino acid-labeled proteins. In one embodiment, the affinity reagent further comprises a second affinity moiety, such as an antigenic moiety that can be recognized by an antibody. In a preferred embodiment, the affinity reagent has a FLAG epitope that can also be used for affinity purification. In a preferred embodiment, the affinity reagent has both a FLAG tag and a biotin moiety. In such embodiments, the affinity moieties may be used independently or in combination (*e.g*., sequential) to effect optimum isolation of the labeled protein through the affinity moieties.

As used herein, "affinity moiety" includes any moiety that may be used for affinity binding / isolation / purification purpose. Common affinity moieties include antigens (*e.g*., epitope tags, such as FLAG tag, HA tag, His6 tag, *etc*.) for certain antibodies, a member of a ligand-receptor pair, *etc*. The affinity moiety may be polypeptide, nucleic acid, polysaccharide, lipids, vitamin (*e.g*., biotin, *etc*.), or molecules of any other chemical nature.

In certain embodiments, the reagent is a fluorescent reagent that comprises the second reactive moiety and one or more fluorescent moieties. Preferably, the fluorescent moiety becomes more fluorescent when the first and the second reactive moieties react with each other, or the unreacted fluorescent reagent can be easily removed (*e*.*g*., washed away).

In certain embodiments, the reagents may be cleavable. In one embodiment, the reagent harbors, in addition to any of the above-described components (such as an antigenic component), a cleavage site for a sequence-specific protease, such as Factor Xa or PreScission Protease. When used as an affinity tag, a first purification can be performed that will select for the presence of the affinity reagent. For example, the proteins can be purified using a FLAG binding resin. Upon treatment with the specific protease, only proteins that have the non-natural amino acid will be released for subsequent analyses, while proteins that bind non-specifically to the affinity reagent or the resin / column will likely stay bound.

In another embodiment, the reagent may comprise a photo-cleavable linker between the second reactive moiety and the functional group. For example, a photo-cleavable affinity reagent includes a photo-labile linker between the second reactive moiety and the one or more affinity moieties. Upon light exposure, non-natural amino acid-bearing molecules are specifically released and can be subsequently identified in various detection assays, such as mass spectrometric analyses.

Proteins or fragments thereof having the non-natural amino acid moiety can be isolated using the one or more affinity moieties, if such moieties are present in the reagent comprising the second reactive moiety. The isolated proteins can be analyzed further. For example, such proteins or fragments can be identified by mass spectrometry techniques, with or without obtaining the sequences of the proteins / fragments, In certain embodiments, the isolated proteins can be digested using a protease, such as trypsin, and the resulting peptides can be analyzed by mass spectrometry. Alternatively, proteins from the cell may be digested with protease before the resulting fragments are reacted with the second reactive groups. The resulting peptide fragment - affinity reagent complex can then be isolated through affinity column. Protein digestion may also be carried out after the reaction with the second reactive moiety, but before the affinity purification / isolation step.

The cells may be treated with a second non-natural amino acid, and/or a natural amino acid derivative. The natural amino acid derivative may contain one or more isotopes such that the overall molecular weight of the amino acid derivative is different from that of a wild-type natural amino acid. For example, the natural amino acid derivative may be a deuterated amino acid (alternatively, an amino acid that contains ³H), which gives a mass shift in mass spectrum. The non-natural amino acid may also contain isotopes, such as ¹³C, ¹⁵N or ¹⁸O. The presence of such isotopes may help to differentiate newly synthesized proteins from different samples, thus enabling simultaneous analysis of multiple samples in, for example, high throughput experiments.

The methods of the disclosure can be used in general to profile the expression pattern of new proteins in two or more samples / cell lines / tissues, and compare such expression patterns.

The methods of the disclosure may be used to study pharmaceutical impact on protein expression in a cell. According to such a method, the protein expression pattern is determined according to the methods of the invention in the presence or absence of a pharmaceutical agent or a candidate drug.

The methods of the disclosure may be used to monitor the changes in expression pattern, if any, of a sample over time. For example, a zero time-point expression pattern may be obtained before the sample is subject to certain treatment. Expression patterns at later time points may be obtained and compared to the zero time point. Pulse labeling of new proteins using the method of the invention may be used to obtain samples from different time points.

The methods of the disclosure can also be used to metabolically label new protein synthesis in cells or tissues. Such method is particularly useful for real-time imaging of local protein synthesis in cells / tissues. For example, in one embodiment, protein synthesis can be monitored within a living cell, such as one in a live organism or in tissue culture. According to such methods, the cell is contacted with a non-natural amino acid, such as AHA, that is incorporated in a protein in place of a naturally occurring amino acid by the cell's endogenous machinery. The cells are then treated with a fluorescent reagent that has a second reactive moiety capable of reacting with the first reactive moiety on the non-natural amino acid residue to form a covalent bond or, alternatively, the cells are treated with a reagent that allows for sequential coupling of a fluorescent moiety. The reaction of the fluorescent tags with the proteins will result in fluorescent-labeled proteins. In a preferred embodiment, the fluorescent reagent is substantially more fluorescent after reaction with the non-natural amino acid residue.

The invention includes reagents for use with the methods of the disclosure. The invention provides an affinity reagent that has a first reactive moiety capable of reacting with a non-natural amino acid, at least one affinity group /moiety, and at least one cleavage site that allows the separation of the second reactive moiety and the affinity moiety. The one or more cleavage sites are selected from photo-cleavable groups; groups which have a recognition sequence for a protease; groups which have a recognition sequence for an endonuclease; and chemically cleavable groups selected from diols, disulfides, diazo groups, esters, sulfones, and groups comprising methionine. In a preferred embodiment, the affinity reagent has two affinity groups and two distinct cleavage sites. In one such embodiment, the affinity reagent has two affinity moieties, including a biotin group and an immunological / epitope tag, and a two peptide cleavage sites (such as two protease cleavage sites for trypsin or trypsin-like proteases).

As used herein, the term "physiological conditions" is meant to encompass those conditions compatible with living cells, *e.g*., predominantly aqueous conditions of a temperature, pH, salinity, etc. that are compatible with living cells.

The term "aryl" as used herein means 5- and 6-membered single-aromatic radicals which may include from zero to four heteroatoms. Representative aryls include phenyl, thienyl, furanyl, pyridinyl, (is)oxazoyl and the like.

The term "lower alkyl", alone or in combination, generally means an acyclic alkyl radical containing from 1 to about 10, preferably from 1 to about 8 carbon atoms and more preferably 1 to about 6 carbon atoms. Examples of such radicals include methyl, ethyl, n-propyl, isopropyl, n-butyl, isobutyl, sec-butyl, tert-butyl, pentyl, iso-amyl, hexyl, octyl and the like.

### 2. Non-natural Amino Acids

Numerous non-natural amino acids have been successfully incorporated into protein either *in vitro* (such as *in vitro* translation) or *in vivo* (*e.g.,* in live cells or animals). These non-natural amino acids usually contain one or more functional groups not present in the twenty naturally occurring amino acids, thus incorporation of non-natural amino acids into proteins *in vivo* can provide biological materials with new chemical functions and improved physical properties. Examples include new posttranslational modification chemistry by introducing azide and ketone moieties into recombinant proteins, and novel strategies for engineering hyper-stable proteins by incorporating fluorinated side chains.

However, implementing such methods generally requires certain manipulation of the protein biosynthesis machinery. This may be accomplished by using a cell's translational machinery in either a residue-specific (Link et al., Curr. Opin. Biotechnol. 14: 603-609, 2003) or a site-specific manner (Wang & Schultz, Angew. Chem. Int. Edn. Engl. 44:34-66, 2004).

Thus residue-specific incorporation of non-natural amino acids into proteins may involve replacement of a natural residue with a conservatively modified analog. The translational machinery is sufficiently tolerant of altered substrates that, especially in the absence of competing natural substrates, the modified residue is converted to an aminoacyl tRNA that is subsequently used by the ribosome. Alternatively, the specificity of certain endogenous or designed / engineered exogenous AARS may be altered to catalyze the attachment of the non-natural amino acids to suitable tRNAs.

By these mechanisms, non-natural amino acids bearing bioorthogonal chemical moieties can be introduced into proteins that are overexpressed in a host cell. To avoid competition with the endogenous amino acid, the host cell (*e.g.,* bacterial strain) may be rendered auxotrophic for the natural amino acid. Proteins cannot be overexpressed unless the cells are supplemented with either that residue or a closely related unnatural analog. For example, a phenylalanine auxotroph was used to express proteins in which all phenylalanine residues were replaced with p-azidophenylalanine or p-acetylphenylalanine (a keto derivative) (Datta et al., J. Am. Chem. Soc. 124: 5652-5653,2002; Kirshenbaum, ChemBioChem 3: 235-237, 2002). Similarly, a methionine auxotroph was used for production of proteins that contained homopropargylglycine or azidohomoalanine at sites that encode for methionine (van Hest, J. Am. Chem. Soc. 122: 1282-1288, 2000; Kiick et al., Proc. Natl. Acad. Sci. USA 99: 19-24, 2002). Applicants have also extended this work to the labeling of bacterial cell surfaces (Link, J. Am. Chem. Soc. 126: 10598-10602, 2004; Link & Tirrell, J. Am. Chem. Soc. 125: 11164-11165,2003). Azido amino acids were installed in outer membrane protein C (OmpC) of an *E*. *coli* methionine auxotroph and the cell surface azides were then ligated with alkyne probes through both copper(I)-mediated and strain-promoted [3+2] cycloaddition.

Residue-specific metabolic labeling can produce proteins with multiple copies of a bioorthogonal functional group, and is particularly useful for the proteome-wise expression profiling. However, this has only limited application in cases where a chemical moiety is desired at a single position within the protein. Thus site-specific insertion of a bioorthogonal amino acid may be achieved using nonsense suppression techniques (Wang & Schultz, Angew. Chem. Int. Edn. Engl. 44: 34-66, 2004). In this approach, a mutually selective tRNA and aminoacyl-tRNA synthetase are developed so that the non-natural amino acid can be uniquely activated by the tRNA *in vivo.* The tRNA's anticodon is engineered to complement a rare stop codon, which is co-opted to encode the non-natural amino acid in the corresponding DNA (and intermediate mRNA). Cells transfected with genes encoding the engineered tRNA, aminoacyl-tRNA synthetase and target protein will produce the modified protein when supplemented with the non-natural amino acid.

The non-natural amino acid mutagenesis method has been used to introduce chemical moieties into proteins in both *E. coli* and yeast. For example, m-acetylphenylalanine was site-specifically incorporated into LamB, an outer-membrane protein of *E. coli,* and subsequently labeled with membrane-impermeant hydrazide dyes (Zhang et al., Bio-chemistry 42: 6735-6746, 2003). Similarly, azido and alkynyl amino acids related to tyrosine were installed in proteins within both *E*. *coli* and yeast. After cell lysis, the derivatized proteins were tagged by copper-catalyzed [3+2] cycloaddition.

Regardless of how the non-natural amino acid is incorporated into proteins, in certain embodiments, it is preferred that the non-natural amino acids contain one or more of the following desirable characteristics to facilitate easy incorporation into live cells *in vitro* or *in vivo*: (1) relatively permeable through bio-membranes, such as plasma membranes, such that it can be directly provided in tissue culture medium or administered to a live organism for direct uptake by the cell; (2) relatively stable *in vitro* and *in vivo*; (3) being a structural homolog of one or more natural amino acids, such as methionine or phenylalanine (In certain embodiments, the natural amino acid is an essential amino acid, so that the cells are auxotrophic for that natural amino acid; in certain embodiments, the natural amino acid is relatively rarely used in proteins); (4) capable of being charged directly to an endogenous tRNA by an endogenous AARS; (5) the charged non-natural amino acid-tRNA complex can be readily incorporated into proteins by endogenous ribosomes; (6) the structure of the non-natural amino acid is such that, upon incorporation into the proteins, it does not substantially affect the folding and/or biological function of any proteins incorporating the non-natural amino acid; and (7) being substantially non-toxic and biologically inert. The non-natural amino acid is typically relatively small in size, and contains a single functional reactive moiety.

The non-natural amino acid may be incorporated in the place of a naturally occurring amino acid. In general, such non-natural amino acid will be an acceptable substrate for an aminoacyl-tRNA synthetase (AARS) that charges a tRNA recognizing a naturally occurring codon. Preferably, the AARS and/or the tRNA are endogenous AARS and endogenous tRNA.

For *in vivo* use, or *in vitro* use in live cells, the non-natural amino acid chosen should not be significantly toxic to cells.

However, the methods of the disclosure also can be utilized in cells, tissues or organisms engineered to express an aminoacyl-tRNA that charges a natural tRNA with the non-natural amino acid. The methods of the disclosure may further be used in *in vitro* translation systems where cell lysates are used to support protein synthesis. In those methods, the requirement of membrane permeability becomes largely irrelevant, and one or more ortholog AARS / tRNA pairs may be provided to the lysate to support incorporation of the non-natural amino acid.

There are several preferred reactive moiety pairs that are particularly suitable for the invention, which are illustrated below as non-limiting examples.

### (A) Azide - Alkyne Ligation

In one specific embodiment, the protein tagging is based on the azide-alkyne ligation. In a typical embodiment, the side chain of the non-natural amino acid contains the azide group, while the second reactive moiety on the reagent contains the terminal alkyne moiety. Alternatively, the side-chain of the non-natural amino acid contains the terminal alkyne, while the second reactive moiety contains the azide moiety.

Several non-natural amino acids contain the azide group and are suitable for the instant invention. For example, azidoalanine, azidohomoalanine (AHA), azidonorvaline, or azidonorleucine are all azide-containing methionine homologs that can be incorporated into proteins using the endogenous protein translation machinery. AHA, which serves as a surrogate for the essential amino acid methionine during protein synthesis, exhibits excellent membrane permeability and is not toxic even in primary neuronal cell cultures. Studies in *E*. *coli* and experiments in mammalian cells have shown no evidence of increased protein degradation upon introduction of AHA, indicating that the modified amino acid is not likely to cause severe protein misfolding.

Azides are suitable for labeling all classes of biomolecules (including proteins) in any biological locale. This versatile functional group is absent from nearly all naturally occurring species. (Only one naturally occurring azido metabolite has been reported to date, isolated from unialgal cultures. See Griffin, Prog. Med. Chem. 31:121-232, 1994). Azides do not react appreciably with water and are resistant to oxidation. Additionally, azides are mild electrophiles that do not react with amines or the other "hard" nucleophiles that are abundant in biological systems. Rather, they require "soft" nucleophiles for reaction. Although azides are susceptible to reduction by free thiols, including the ubiquitous cellular reductant, glutathione, reactions between monothiols and alkyl azides typically require vigorous heating (100°C for several hours) or auxiliary catalysts.

Azides are prone to decomposition at elevated temperatures, but they are quite stable at physiological temperatures (See Griffon, Prog. Med. Chem. 31: 121-232, 1994). Whereas aryl azides are well-known photocrosslinkers, alkyl azides do not photodecompose in the presence of ambient light. Finally, although azide anion (for example, in the form of NaN₃) is a widely used cytotoxin, organic azides have no intrinsic toxicity. Indeed, organic azides are components of clinically approved drugs such as AZT (See Griffin, Prog. Med. Chem. 31: 121-232, 1994).

Although kinetically stable, azides are predisposed to unique modes of reactivity owing to their large intrinsic energy content. This feature has been exploited for the development of bioorthogonal reactions, including the Staudinger ligation of azides with functionalized phosphines and the [3+2] cycloaddition of azides with activated alkynes. These reactions can be used for the selective labeling of azide-functionalized biomolecules.

**Staudinger ligation**. In 1919, Hermann Staudinger reported that azides react with triphenylphosphines (soft nucleophiles) under mild conditions to produce aza-ylide intermediates (Staudinger & Meyer, Helv. Chim. Acta 2: 635-646, 1919). Bertozzi *et al*. modified the classic Staudinger reaction by introduction of an intramolecular trap into the phosphine (Saxon & Bertozzi, Science 287: 2007-2010, 2000). Now known as the Staudinger ligation, this transformation ultimately produces a covalent link between one nitrogen atom of the azide and the triarylphosphine scaffold (see below).

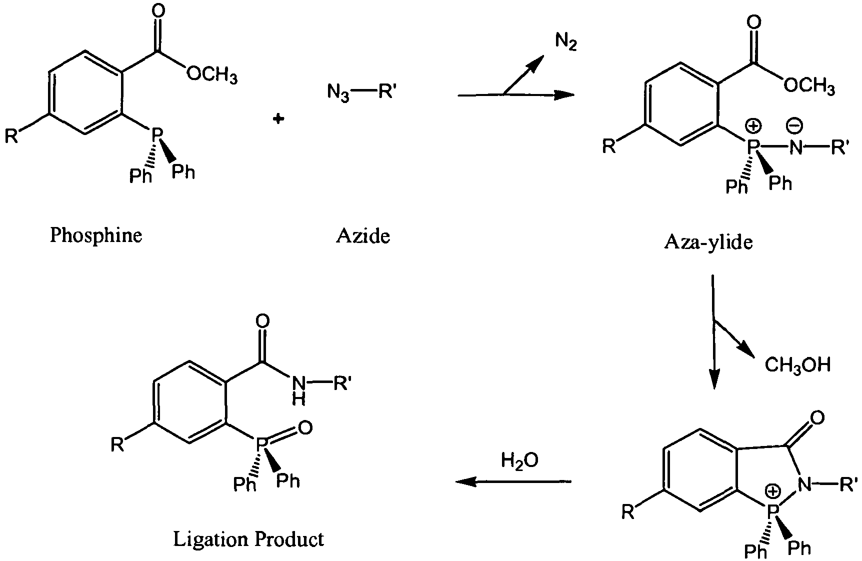

### The Staudinger Ligation

The Staudinger ligation can be used to covalently attach probes to azide-bearing biomolecules. Like the azide, phosphines do not react appreciably with biological functional groups and are therefore also bioorthogonal. Additionally, the reaction proceeds readily at pH 7 with no apparent toxic effects. These and other applications of the Staudinger ligation have been recently reviewed (see Kohn & Breinbauer, The Staudinger ligation - a gift to chemical biology. Angew. Chem. Int. Edn. Engl. 43: 3106-3116, 2004).

**Copper-catalyzed [3+2] azide-alkyne cycloaddition**. In the context of the Staudinger ligation, the azide serves as an electrophile subject to reaction with soft nucleophiles. Azides are also 1,3-dipoles that can undergo reactions with dipolarophiles such as activated alkynes. These π-systems are both extremely rare and inert in biological systems, further enhancing the bioorthogonality of the azide along this reaction trajectory.

The [3+2] cycloaddition between azides and terminal alkynes to provide stable triazole adducts was first described by Huisgen more than four decades ago (Huisgen, Angew. Chem. Int. Edn. Engl. 2; 565-598, 1963). The reaction is thermodynamically favorable by 30-35 kcal/mol. The reaction typically requires alkyne activation, which process requires elevated temperatures or pressures that are not compatible with living systems. Although this may not be a concern for *in vitro* use, activation may be achieved with catalysts for use *in vitro* as well as *in vivo.*

In one embodiment, the activation may be achieved by the addition of electron-withdrawing groups, such as esters, to the alkyne.

In another embodiment, a Cu(I)-based catalyst may be used to accelerate the rate of cycloaddition between azides and alkynes by about 10⁶-fold (Rostovtsev et al., Angew. Chem. Int. Edn. Engl. 41: 2596-2599, 2002; Tomoe, J. Org. Chem. 67: 3057-3064,2002). This copper-catalyzed reaction, termed "click" chemistry, proceeds readily at physiological temperatures and in the presence of biological materials to provide 1,4-disubstituted triazoles with nearly complete regioselectivity (Kolb & Sharpless, Drug Discov, Today 8: 1128-1137, 2003). The copper-mediated reaction has been used to tag azides installed within virus particles (Wang et al., J. Am. Chem. Soc. 125: 3192-3193,2003), nucleic acids (Seo et al., Proc. Natl. Acad. Sci. USA 101: 5488-5493, 2004) and proteins from complex tissue lysates (Speers & Cravatt, ChemBioChem 5: 41-47, 2004) with virtually no background labeling.

It should be noted that the same reaction can be carried out in reverse - using the alkyne as the first reactive moiety on the non-natural amino acid. Like the azide, a terminal alkyne consists of a mere three atoms.

The primary advantage of the catalyzed azide-alkyne cycloaddition over the Staudinger ligation is its faster rate. The copper-catalyzed reaction of azides with alkynes reportedly proceeds at least 25 times faster than the reaction of azides with triarylphosphines in cell lysates. Accordingly, "click" chemistry is preferably used in situations that require detection of very small quantities of azide-labeled biomolecules.

In certain embodiments, an improved protocol for copper-catalyzed triazole formation may be used to further increase the efficiency of reaction by about 10-fold (Link et al., J. Am. Chem. Soc. 126: 10598 - 10602, 2004.

According to this embodiment of the invention, instead of generating Cu(I) *in situ* by combining Cu(II) and a reducing agent (such as 200 µM CuSO₄ and 200 µM of tris-(carboxyethyl)phosphine (TCEP)), ultra-pure Cu(I) is added directly to the reaction mixture as catalyst. In a preferred embodiment, the ultra-pure Cu(I) is CuBr, with at least 99.999% purity (such as those obtained from Aldrich).

**Strain-promoted cycloaddition**. An alternative means of activating alkynes for catalyst-free [3+2] cycloaddition with azides involves the use of ring strain (Agard et al., J. Am. Chem. Soc. 126: 15046-15047, 2004). Constraining the alkyne within an eight-membered ring creates ~18 kcal/mol of strain, much of which is released in the transition state upon [3+2] cycloaddition with an azide (Turner et al., J. Am. Chem. Soc. 95: 790-792, 1972). As a consequence, cyclooctynes react with azides at room temperature, without the need for a catalyst. This strain-promoted cycloaddition has been used to label biomolecules both *in vitro* and on cell surfaces without observable toxic effects (Agard et al., J. Am. Chem. Soc. 126: 15046-15047, 2004). The rate of the strain-promoted cycloaddition can be increased by appending electron-withdrawing groups to the octyne ring.

### (B) Ketones and Aldehydes

A second group of non-natural amino acids that can be incorporated into proteins according to the method of the instant invention includes those with a ketone or aldehyde side chain. Comprising only a handful of atoms, ketones and aldehydes are bioorthogonal chemical moieties that can tag not only proteins, but also glycols and other secondary metabolites. These mild electrophiles are attractive choices for modifying biomolecules as they are readily introduced into diverse scaffolds, absent from endogenous biopolymers and essentially inert to the reactive moieties normally found in proteins, lipids and other macromolecules. Although these carbonyl compounds can form reversible Stiff bases with primary amines such as lysine side chains, the equilibrium in water favors the carbonyl. By contrast, the stabilized Stiff bases with hydrazide and amino groups (hydrazones and oxides, respectively) are favored in water and are quite stable under physiological conditions (Jencks, J. Am. Chem. Soc. 81: 475-481, 1959).

In one embodiment, ketone (and aldehyde) condensations are used for chemical modifications in the presence of cultured cells.

Ketone (and aldehyde) condensations may be used to label proteins on cell surfaces or in the extra cellular environment.

Ketone (and aldehyde) condensations may be used in the context of selectively labeling tissues in living organisms.

### 3. Reagents with Second Reactive Moiety

The reagents with the second reactive moiety comprise at least two moieties: a second reactive group that can specifically react with the first reactive moiety of the non-natural amino acid, and a functional moiety that becomes attached to the incorporated non-natural amino acid after the reaction. Optionally, a cleavable functional group is situated between the second reactive moiety and the functional moiety to allow separation of these two moieties under controlled conditions (e.g., protease digestion, photo-cleavage, etc.).

A number of possible functional moieties can be linked to the second reactive moiety. For example, the functional moiety may be an affinity moiety that can be used to isolate the labeled proteins or fragments thereof.

Alternatively, any other functional groups, such as a radioactive moiety (*e.g*., radioisotope), a fluorescent moiety, *etc*., may also be attached to the labeled protein or fragments thereof containing the non-natural amino acids.

### (A) Second Reactive Moiety

In one embodiment, when the non-natural amino acid used is AHA or another azide-bearing non-natural amino acid, proteins bearing one or more azide groups can subsequently be tagged for enrichment by treatment with an alkyne-bearing affinity reagent. Alternatively, as described above, the affinity reagent can have a phosphine group for reacting with the azide moiety via a Staudinger reaction (Saxon and Bertozzi, Cell Surface Engineering by a Modified Staudinger Reaction, Science 287: 2007-2010, 2000.

As used herein, "azide" group refers to R-N₃, wherein R represents the rest of the azide-containing molecule (comprising the non-natural amino acid side chain).

"Terminal alkyne" refers to R'-C≡C, wherein the [3+2] cycloaddition product of the R-N₃ + R'-C≡C reaction has a general formula of:

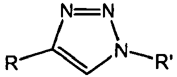

When "strain-promoted cycloaddition is used, the terminal alkyne in the above reaction is replaced by the following reactive partner:

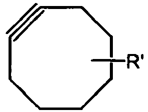

In this case, the final ligation product of the cycloaddition is:

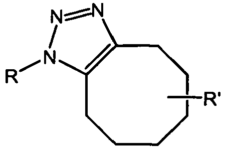

In other embodiments, where the non-natural amino-acid has a ketone group, the affinity reagent will preferably have an aminooxy or hydrazide moiety (NH₂-NH-CO-R') (See, for example, U.S. Pat. Appln. No. 20020016003 to Bertozzi).

As used herein, "ketone" group refers to the general formula: R-CO-R₂; "aldehyde" group refers to the general formula R-CHO.

"Aminooxy" moiety refers to NH₂-O-R'. The reaction product between the aminooxy group and the ketone / aldehyde group is:

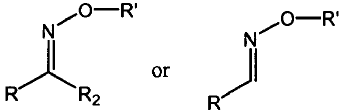

### (B) Functional Moiety

In one embodiment, the functional moiety is an affinity moiety, and thus the reagent is an affinity reagent. The affinity reagent has at least one affinity moiety that can be used to purify labeled proteins. In one embodiment, the affinity group is a biotin moiety. In another embodiment, the affinity group is an immunological or epitope tag. In a preferred embodiment, the affinity reagent has two or more affinity groups, thus allowing tandem purification of the labeled proteins. Tandem purification decreases contamination by proteins that non-specifically adhere to the matrices or support material (such as resin) used for purification.

The affinity reagent can have one or more cleavable sites that allow for release of the purified proteins from the affinity matrix. The cleavable sites can be peptide bonds cleavable by proteases, or can be chemical cleavage or photo-labile sites built into the affinity reagent. In a preferred embodiment, the cleavage sites are peptide sequences that can be specifically cleaved by proteases such as trypsin.

In certain embodiments, the cleavable sites are at or adjacent to the epitope tag. For example, in Figure 2, the FLAG tag sequence contains two trypsin cleavage sites that allow for the cleavage of the FLAG sequence, thereby releasing the attached protein or fragment incorporating the non-natural amino acid.

Other exemplary functional moieties include, but are not necessarily limited to, fluorescent molecules or tags (*e.g*., auto-fluorescent molecules, molecules that fluoresce upon contact with a reagent, etc.), radioactive labels (e.g., ¹¹¹In, ¹²⁵I, ¹³¹I, ²¹²B, ⁹⁰Y, ¹⁸⁶Rh, and the like); biotin (*e*.*g*., to be detected through reaction of biotin and avidin); imaging reagents (*e.g*., those described in U.S. Pat. No. 4,741,900 and U.S. Pat. No. 5,326,856), and the like. Functional moieties may also include peptides or polypeptides that can be detected by antibody binding, e.g., by binding of a detectably labeled antibody or by detection of bound antibody through a sandwich-type assay.

In one embodiment, an engineered phosphine may act as an azide-selective phosphine dye. In this embodiment, the dye remains substantially undetectable until reaction with an azide. In general, the phosphorous lone pair renders the dye substantially undetectable (*e*.*g*., substantially non-fluorescent) until reaction with an azide, when the lone pair is removed from conjugation by formation of a phosphine oxide. The unmasked dye provides for a detectable signal following reaction with the azide of the target molecule according to the invention. This reaction is described in detail in paragraphs [0078] - [0085] of US 2002-0016003 A1. Briefly, the masked dye generally comprises an aryl group substituted with R₁ and PR₂R₃; wherein R₁ is an electrophonic group to trap (e.g., stabilize) an aza-ylide group, including, but not necessarily limited to, a carboxylic acid, an ester (e.g., alkyl ester (e.g., lower alkyl ester, benzyl ester), aryl ester, substituted aryl ester), aldehyde, amide, e.g., alkyl amide (e.g., lower alkyl amide), aryl amide, alkyl halide (e.g., lower alkyl halide), hoister, colony ester, alkyl ketone (e.g., lower alkyl ketone), aryl ketone, substituted aryl ketone, halosulfonyl, nitrile, nitro and the like; R₂ and R₃ are generally aryl groups, including substituted aryl groups, or cycloalkyl groups (e.g., cyclohexyl groups) where R₂ and R₃ may be the same or different, preferably the same; R₁ and PR₂R₃ are preferably in the ortho position relative to one another on an aryl ring of the dye, or in an equivalent position that provides for the reaction to proceed according to the invention; X represents a target molecule having an azide N3; and where R₁ represents a modified R₁ group following reaction with the azide of the target molecule via the Staudinger ligation reaction described herein.

In one exemplary embodiment, the phosphine dye is a fluorescein derivative, which may be in unacetylated or acetylated (cell permeable) form. Three phosphine dyes are described in detail in paragraphs [0086] - [0089] of US 2002-0016003 A1. These phosphine dyes can be used to detect an azide on any molecule, such as those proteins incorporating AHA. The phosphine dyes can be used to detected biomolecules having an azide either at the cell surface or within cells.

### (C) Cleavable Functional Group

The reagents with the second reactive moiety may optionally contain one or more cleavable functional groups to allow separation of the second reactive moiety and the functional moiety under one or more controlled conditions. The functional reagents of claim 1 do contain one or more cleavable groups as defined in the claim.

As used herein, a "cleavable functional group" or "cleavable linker" is a chemical group that can be cleaved by a variety of methods, including input of energy, a chemical, an enzyme, and the like. For use in methods of the invention, the cleavable functional group is generally specific, that is, one which can be specifically cleaved without altering or damaging the molecule being cleaved or which relatively uniformly alters the molecule in a reproducible manner. For example, the cleavable functional group can be a photo-cleavable group. In such a case, the photo-cleavable group is generally cleaved at a wavelength of light that does not damage the molecule being released, for example, in the ultraviolet to visible range. Exemplary photo-cleavable linkers include, for example, linkers containing o-nitrobenzyl, desyl, trans-cinnamoyl, m-nitrophenyl, benzylsulfonyl groups and the like (see, for example, Dorman and Prestwich, Trends Biotech. 18: 64-77, 2000; Greene and Buts, "Protective Groups," in Organic Synthesis, 2nd ed., John Wiley & Sons, New York, 1991; U.S. Patent Nos. 5,143,854; 5,986,076; 5,917,016; 5,489,678; 5,405,783).

The cleavable functional group can also be a chemically cleavable group cleavable by a chemical such as an acid or base. If desired, a chemically cleavage reaction can be carried out under relatively mild conditions in which the chemically cleavable group is essentially the only chemical bond cleaved. A chemically cleavable group can also be a group cleavable by a chemical such as CNBr, which can cleave a methionine residue. CNBr can be particularly useful for releasing a molecule if a chemically cleavable group such as methionine has been added to the molecule, particularly in a polypeptide that does not have a methionine residue, or when the methionine residues have been replaced by non-natural amino acids.

Suitable chemically cleavable groups are well known to those skilled in the art (see, for example Wilson and Czarnik, eds., Combinatorial Chemistry: Synthesis and Application, John Wiley & Sons, New York, 1997; Merrifield, J. Am. Chem. Soc. 85: 2149, 1964; Bodanszky, Principles of Peptide Synthesis, Springer-Verlag, 1984; Houghten, Proc. Natl. Acad. Sci., USA 82: 5131, 1985). Exemplary chemically cleavable linkers can contain a disulfide, which can be cleaved with reducing agents; a diol, which can be cleaved with periodate; a diazo bond, which can be cleaved with dithionate; an ester, which can be cleaved with hydroxylamine; a sulfone, which can be cleaved with base, and the like (see Hermanson, Bioconjugate Techniques, Academic Press, San Diego, 1996; Pierce Chemical Co., Rockford IL).

The cleavable functional group can also be an enzymatically cleavable group. For example, a protease can be used to cleave a cleavable functional group having a suitable recognition sequence for the protease.

Particularly useful proteases are endopeptidases such as factor Xa, tobacco etch virus (TEV) protease, trypsin, chymotrypsin, *Staphylococcus aureus* protease, submaxillaris protease, and the like. The protease can be selected based on the incorporation of a particular cleavable recognition sequence as a functional group. Other considerations for selecting a protease include the presence or absence of a recognition sequence in the molecule being captured and released. For example, a rare cleaving protease such as TEV protease or factor Xa can be used to cleave a functional group containing the corresponding protease recognition sequence, resulting in release of the captured molecule.

Such rare cleaving proteases are particularly useful for releasing an intact polypeptide molecule since the recognition sequence for these proteases would not occur in the vast majority of polypeptides. Alternatively, a polypeptide sample can be treated with a specific protease, and the digested peptides isolated by the methods disclosed herein. In such a case, the captured peptides would not contain a recognition sequence for the protease used for cleavage since the polypeptide has already been digested.

In addition, in certain embodiments, an intact polypeptide can be captured and digested with a protease after binding to the solid support, resulting in the incorporation and release of a label on the peptide fragment of the polypeptide that was captured on the solid support. Thus, protease digestion can be used before or after capture of a sample molecule, in particular polypeptide sample molecules, as desired.

In addition to proteases, a cleavable functional group can be a recognition sequence for an endonuclease such as a restriction enzyme. Thus, an appropriate recognition sequence for a restriction enzyme can be incorporated as a cleavable functional group and cleaved with the respective restriction enzyme. It is understood that such a nucleotide functional group can be useful for capturing and releasing a nucleic acid or a polypeptide, or any other type of molecule, as desired. Similarly, a protease recognition sequence can be useful for capturing and releasing a polypeptide, nucleic acid or any other type of molecule, as desired.

As used herein, the term "isotopic label" or "isotope tag" refers to a chemical group which can be generated in two distinct isotopic forms, for example, heavy and light isotopic versions of the constituent elements making up the chemical group. Such constituent elements include, for example, carbon (e.g., ¹³C or ¹⁴C), oxygen (*e.g.,* ¹⁸O), hydrogen (*e.g.*, ²H or ³H), nitrogen (*e.g.*, ¹⁵N), and sulfur (*e.g*., ³⁵S). In addition, other elements that are chemically or functionally similar can be substituted for the above naturally occurring elements. For example, selenium can be used as a substitute for sulfur. Particularly useful isotopic labels or tags are those that allow convenient analysis by MS. In such cases, the isotope is preferably nonradioactive or with low radioactivity. For example, heavy and light isotopic versions of an amino acid can be used to differentially isotopically label a polypeptide.

### 4. Detection and Analysis

After tryptic digestion, mass spectral analysis may be used to analyze the eluted non-natural amino acid-containing proteins or fragments thereof.

The eluted proteins may be analyzed using conventional techniques, such as 2-D electrophoresis.

Detection and analysis may be achieved by utilizing MudPIT (Multi-dimensional Protein Identification Technology) or related advanced mass spectrometrical techniques. MudPIT is a technique for the separation and identification of complex protein and peptide mixtures. MudPIT is another approach developed to alleviate many of the disadvantages associated with two-dimensional gel electrophoresis. MudPIT uses two chromatography steps interfaced back to back in a fused silica capillary. More specifically, MudPIT uses columns consisting of strong cation exchange (SCX) material back-to-back with reversed phase (RP) material inside fused silica capillaries. The chromatography proceeds in cycles, each comprising an increase in salt concentration to "bump" peptides off of the SCX followed by a gradient of increasing hydrophobicity to progressively elute peptides from the RP into the ion source. In other words, chromatography proceeds in steps with increases in salt concentration used to free peptides from the cation-exchange resin, after which they bind to a reversed phase resin. A typical reversed phase gradient to increasing hydrophobicity is then applied to progressively elute peptides from the reversed phase packing into the mass spectrometer. Typically, this mass spectrometer will be a tandem electrospray (ESI-MS/MS), so peptides are ionized in the liquid phase, separated in a primary mass spectrometer, broken up using collision-induced dissociation (CID) and analyzed again.

The advantage of this process is that the band broadening associated with many chromatographic steps is avoided, thus avoiding loss of resolution, and preventing components from running into one another. In addition, the capillary can be placed directly into the ion source of a mass spectrometer to maximize sensitivity.

Raw mass spectral data are filtered and analyzed using art-recognized methods, such as SEQUEST, DTASelect and Contrast algorithms, which allow for an efficient and comprehensive interpretation and comparison of the proteomic data. Specifically, the mass spectrometer's data-dependent acquisition isolates peptides as they elute, and subjects them to Collision-Induced Dissociation, recording the fragment ions in a tandem mass spectrum. These spectra are matched to database peptide sequences by the SEQUEST algorithm. SEQUEST's peptide identifications are assembled and filtered into protein-level information by the DTASelect algorithm.

In a typical setting, a Sutter Instrument Company P-2000 is used to produce tips from fused silica capillaries, which are usually purchased from Polymicro or Agilent. Apertures are approximately 5 nanometers across from capillaries that have an inner diameter of 100 microns. The material that is loaded into the SCX columns can be made from 5 micron spherical silica beads. For RP, 5 micron C-18 coated beads from a variety of commercial vendors can be used. Agilent 1100 and ThermoFinnigan Surveyor Quaternary pumps may be operated at flow rates of 100-200 microliter/min, with pre-column splitting of the flow to produce about 100-200 nL/min flow rates at the column.

SEQUEST is a program that correlates uninterpreted tandem mass spectra of peptides with amino acid sequences from protein and nucleotide databases. SEQUEST will determine the amino acid sequence and thus the protein(s) and organism(s) that correspond to the mass spectrum being analyzed. SEQUEST is distributed by Thermo Finnigan, A division of Thermo Electron Corporation. SEQUEST uses algorithms described in U.S. patents 6,017,693 and 5,538,897.

While SEQUEST is very powerful for matching uninterpreted tandem mass spectra to database peptide sequences, DTASelect is designed to reassemble this peptide information into usable protein information. The DTASelect program can do more than simply report the protein content of a sample; it features many customizable filters to specify which identifications should be kept and which discarded. It also features reports to investigate post-translational modifications, align sequences of peptides to identify poorly sequenced regions, and analyzed chromatography efficiency. The software makes the process of analyzing SEQUEST results much faster, more powerful, and more consistent than possible before, even for data sets containing a million spectra or more. By automating SEQUEST analysis, DTASelect enables experiments of far greater scope. Alternatively, programs like PeptideProphet and ProteinProphet can be used for data processing after the SEQUEST algorithm. Programs mentioned above and in the following paragraph are only examples for performing the data analysis.

Differentiating biological samples by protein content is an important application of proteomics. The CONTRAST program uses the filters present in DTASelect to highlight the most important identifications of samples and then compares them. Unlike most relatively simple comparison algorithms, Contrast can differentiate up to 63 different samples at once. Contrast handles differential analysis between experimental and control samples simply and flexibly.

Both the DTASelect and the Contrast programs can be licensed for use from The Scripps Research Institute (La jolla, CA). For reference, see Tabb et al., DTASelect and Contrast: Tools for Assembling and Comparing Protein Identification from Shotgun Proteomics. J. Proteome Res. 1: 21-26, 2002.

### 5. Exemplary Uses

To exclude contamination by somatically synthesized proteins, newly synthesized proteins from either synaptoneurosomes or isolated dendrites of hippocampal cultures can be analyzed. Isolated dendrites are obtained from a special culture system using polycarbonate nets to separate dendrites from cell bodies. Individual datasets of newly synthesized proteins can be obtained following different parameters of synaptic stimulation. Once compared, these datasets provide a detailed picture of the local molecular changes associated with different patterns of synaptic activity or the presence or absence of different neuromodulators. Furthermore, the approach allows the applicants to assess the general translational capacity of dendrites by quantifying single proteins in comparison to their ¹⁵N-substituted counterparts. Verification of the data can be examined at the mRNA level (in situ hybridization, RT-PCR) as well as at the protein level (immunocytochemistry and immunoprecipitation studies). The use of cell permeable, fluorescent alkyne-tags further expands this technology to *in vivo* real-time imaging of local protein synthesis in small dendritic segments by restricted application of labeling reagents and neuromodulators. This helps to address the question of whether spatial specificity of locally synthesized proteins are locally synthesized proteins delivered only to the primary site of activation, or are they delivered to neighboring synapses as well.

Other possible applications, for instance, include proteomic profiling of transgenic animals in comparison to their wild-type littermates or profiling of pathological states and changes during development of a tissue and even a whole organism. Knowing the proteomic differences of two cellular compartments, like somata and dendrites, or a brain region challenged either by a missing particular gene or a neuropathological state, helps to understand the molecular and subcellular mechanisms of learning and memory formation, and of neurodegenerative diseases.

The invention circumvents the various problems associated with traditional approaches, by providing an approach to protein identification that unites techniques from organic chemistry, high throughput mass spectrometry and bioinformatics. For example, the procedure can be used to label newly synthesized dendritic proteins.

### Examples

The following examples are for illustrative purpose only, and should in no way be construed to be limiting in any respect of the claimed invention.

### Example 1 Culturing Neurons

Dissociated hippocampal neuron cultures were prepared from newborn rat pups (PO) as outlined in (Banker and Goslin, 1990), Neurons were plated at a density of 15,000 - 45,000 cells/cm² onto poly-D-lysine coated cell culture dishes or onto poly-D-lysine and growth factor reduced matrigel (BD Biosciences) coated polycarbonate nets with a pore size of 3 µm (Transwell, Coming) for the preparation of isolated dendrites. The cultures were maintained and allowed to mature in growth medium (Neurobasal-A supplemented with B27 and GlutaMAX-1) for 14 to 21 days before use. The use of this growth medium suppressed glial proliferation, which was even more reduced by application of Ara-C (5 µM final concentration). To supplement glial neurotrophic factors, 25% conditioned growth media from glial and cortical cultures were added to the growth media. Isolated dendrites were obtained from Transwell cultures by removing the cell body layer with a sterile cell lifter after change to HBS. For immunolabeling after AHA-incorporation and cycloaddition with BiotinCyclooctyne, neurons are fixed at room temperature with 4% paraformaldehyde for 20 minutes. Fixed cultures are then treated sequentially with PBS, blocking solution (0.2% Triton X-100,2 mg/ml BSA, 5% Sucrose, 10% normal horse serum in PBS), primary Ab in blocking solution at 4°C overnight, PBS-Tx (0.2% Triton X-100 in PBS), Alexa488- or Alexa568-conjugated secondary Ab in blocking solution, PBS-Tx and PBS. Immunostained specimens are imaged in PBS with an Olympus confocal microscope or two-photon microscope through either a 10 x or a 63 x oil-immersion lens. Alexa568 is excited at 543 nm and Alexa488 at 488 nm. Images are recorded through standard emission filters at contrast settings for which the crossover between the two channels is negligible.

### Example 2 [3+2] Cycloaddition Chemistry and Purification of Tagged Proteins

In embodiments using [3+2] Cycloaddition, the following exemplary protocol may be used. Briefly, AHA, Biotin-PEO-Propargylamide and the triazole ligand were prepared as described previously [3, 46, 47]. The tandem featured alkyne tag was synthesized by GenScript Corporation. Biotin-Cyclooctyne is a generous gift from Carolyn Bertozzi [45]. D10-L-leucine was purchased from Sigma. In all culture experiments, growth medium was removed from HEK293 cells, whole neuronal cultures or isolated dendrites were replaced with HEPES-buffered solution (HBS) [48] with 2.86 mM AHA, and 2.86 mM methionine for control experiments. After incubation at 37°C, 5% CO₂, cells were washed with PBS-MC (1 mM MgCl₂, 0.1 mM CaCl₂ in PBS) to remove excess amounts of ABA and methionine. SNS and other biochemical fractions are incubated with 2.86 mM AHA or 2.86 mM methionine under agitation at 37°C and washed in StimBuffer after incubation. Cells and fractions are lysed in 0.5% SDS, 1% Triton X-100 in PBS, with DNA destroyed by syringe and needle use. Lysates are diluted to 0.1% SDS, 0.2% Triton X-100 in PBS, complete protease inhibitor and treated for 10 min at 70°C. After addition of 200 µM triazole ligand, 50 µM alkyne tag and 75 µg/ml CuBr, the reaction is allowed to proceed for 16 hours at 4°C. After conclusion of the reaction, samples are dialyzed in 0.2% Triton X-100, 1 mM EDTA in PBS to remove excess reagents. Tagged proteins are purified using monomeric avidin columns followed by FLAG-M2-antibody immunoprecipitations. Eluted proteins are denatured in 8M Urea and proteolytically digested with endoproteinase Lys-C and trypsin (both Roche) as described in [49].

### Example 3 Improved Cu(I) Catalysis

An aliquot of cells expressing recombinant OmpC (1 mL) was centrifuged at 4°C and washed once in 1 mL of PBS (pH 7.4). The cells were centrifuged and resuspended in 1 mL of PBS. Triazole ligand 5 was added to a final concentration of 200 µM, and biotin-PEO-propargylamide 6 was added to a final concentration of 50 µM. Addition of the active copper species was accomplished in two different ways. For in situ generation of Cu(I), 100 µM CuSO₄ and 200 µM of tris-(carboxyethyl)phosphine (TCEP) were added to the cells. Alternatively, the Cu(I) ion was added directly to the cells in the form of an aqueous suspension of CuBr. Briefly, 10 µL of a 10 mM suspension of CuBr (99.999% purity, Aldrich) was thoroughly agitated and added to the cells. As discussed in the Results section, the quality of the CuBr is critical for the success of the experiment. All labeling reactions were allowed to continue for 16 h at 4°C and were stopped by washing the cells with PBS.

### Example 4 Mass Spectrometric Analysis

Analysis of peptide mixtures by MudPIT was done essentially as described in Graumann *et al.* [49] using a LCQ-DecaXP electrospray ion trap mass spectrometer (ThermoElectron). Raw mass spectra were filtered and analyzed with 2to3, SEQUEST, DTASelect and Contrast algorithms [33,34] using standard and modification settings to find modified amino acid residues, like AHA in its possible tagged (+107 mass units) or failed-tagged (-5.1 mass units) version or D10-deuterated 1-leucine (+10 mass units).

The current state-of-the-art tandem mass spectrometry approaches, such as MudPIT, couple in-line multidimensional chromatographic separations with continuous acquisition of data from a column effluent, allow collecting tens of thousands of spectra in a single experiment. Raw mass spectral data were filtered and analyzed using 2to3, SEQUEST, DTASelect and Contrast algorithms [33,34], which validate the data and allow for efficient and comprehensive interpretation and comparison with proteomic databases. In addition to the validation parameters set by the algorithms, the incorporation of stable isotope amino acids, for instance a deuterated variant of L-leucine, can serve as another inner validation for newly synthesized proteins [35]. Certain non-limiting exemplary data addressing the above issues are included in the following section.

### Example 5 Pharmacology

In pharmacology experiments, the following drugs will be applied at the following concentrations: CNQX: 10 µM (Sigma); APV: 50 µM (Sigma); Anisomycin: 40 µM (Sigma); Cycloheximide: 107 µM (Sigma); DHPG: 50 µM (Tocris); NMDA: 50 µM (Sigma); BDNF: 50 ng/ml (Promega); MG132: 50 µM; glutamate/glycine: 100 µM/10 µM (Sigma), KCl:90 mM (Sigma).

### Example 6 Use of a Non-canonical Amino Acid AHA to Label and Identify Newly Synthesized Proteins

This example demonstrates the general suitability of the invention for mammalian cells, such as HEK293 cells and dissociated hippocampal neurons. To assess the purification of a target protein, cells were either transfected (HEK293 cells) or infected (dissociated hippocampal neurons) with a construct coding for a destabilized GFP protein. For neuronal infections, the destabilized and myristoylated GFP reporter described in Aakalu *et al.* [4] is used. Meanwhile, HEK293 cells are transfected with the pd2EGFP-N1 plasmid (Clontech) using PolyFect (Qiagen). The use of GFP enables monitoring protein levels in intact cells using fluorescence, as well as monitoring protein levels in cell lysates using Western blot analysis.

Figure 1 is a general overview for the suitability of AHA for use in the methods of the invention. Applicants first examined the specificity of AHA incorporation, and its potential toxicity, using whole neuron morphological measurements as well as measurements of protein degradation. Incubation with methionine was used as a general control in all of the performed experiments. In order to visualize their gross morphological structure, neurons were infected with a destabilized form of the fluorescent protein EGFP, and incubated for 1.5 hours with the same molar concentration of either AHA or methionine.

Dissociated hippocampal cultured neurons (12 DIV) were infected with a destabilized and myristoylated variant of GFP, whose mRNA is targeted into dendrites ([4]). Nine hours post infection, cells were incubated for 2 hrs with equimolar concentrations of AHA or methionine. Neurons expressing the GFP reporter indicating no change in the gross morphology of AHA-incubated neurons compared to methionine controls. Thus AHA is not toxic to neurons, as evidenced by intact neuronal processes and continuous non-blebby expression of GFP throughout the dendritic arbor.

Applicants also confirmed the successful incorporation of AHA into proteins in these cultures by subsequent biotinylation with the alkyne linker and Western blot analysis. Expressed GFP was selectively enriched by avidin chromatography from AHA-treated cells as compared to methionine-treated cells. A Western blot of avidin-purified samples was performed using either anti-biotin-antibody or an anti-GFP antibody. In both experiments, the eluate was enriched for the presence of biotin-labeled proteins, indicating that the purification was successful.

To show the specificity of this technique for the detection of newly synthesized proteins, HEK293 cells were incubated for 2 hrs with AHA in the presence or absence of a protein synthesis inhibitor (Anisomycin or Cycloheximide). After incubation, cells were lysed and subjected to [3+2]cycloaddition with the biotin-bearing alkyne-linker Biotin-PEO-Propargylamide [3], followed by Western blot analysis with an anti-Biotin-antibody. No signal was detected in either the methionine or the protein synthesis inhibitor lanes, indicating the specificity of this technique as well as the membrane permeability of the reagent. The potential for increased protein degradation was measured by examining the ubiquitin signal strength on a Western blot from whole lysates of BEK293 cells, which were treated with AHA, methionine or incubation buffer for two hours. No increased ubiquitination was observed in AHA treated cells compared to buffer or methionine controls, indicating that the modified amino acid does not cause severe protein misfolding.

### Example 7 Purification of AHA-labeled (Biotinylated) Proteins

Following protein synthesis, cell lysates were subjected to a [3 + 2]-cycloaddition reaction with a biotin-bearing alkyne linker. To initially address our ability to purify AHA-labeled proteins, we have conducted avidin-affinity chromatography of biotinylated GFP, which was expressed in HEK293 cells or neuronal cultures.

In the same system, biotinylated GFP was detected in the eluate of a GFP-immunoprecipitation from AHA-treated cells followed by cycloaddition with the biotin-alkyne tag, but was not detected in methionine-treated control cells. Biotinylated GFP can be immunoprecipitated with a GFP-antibody from AHA-treated neuronal cultures or HEK293 cells, but not from methionine treated cultures.

### Example 8 Mass Spectrometry

About 200 proteins were identified and validated in a tandem mass spectrometry analysis of single avidin-purified proteins from AHA-treated whole cell lysates of HEK293 cells. In this experiment, identifications for a protein were considered as valid, if at least two fully tryptic peptides were assigned to a protein, with at least one peptide harboring a modification due to AHA or a deuterated leucine.

### Example 9 An Affinity Reagent with Two Affinity Groups

To increase the specificity of the initial purification of tagged proteins by a two-step procedure. Applicants have developed an affinity reagent which allows for a two-step purification. One goal is to diminish contamination by proteins that adhere non-specifically to the matrices used for affinity-purification. In addition, a tag is introduced, which tag is suitable for detection by mass spectral analysis, and therefore can be used as an inner validation of the data.

The first alkyne tag used in the development of the technique, Biotin-PEO-Propargylamide, resulted in a gain of 578 mass units per reaction and was difficult to detect in the analysis because of its high molecular weight. To increase the purification of the modified proteins and facilitate detection by mass spectrometry, we have designed a new alkyne tag (Fig 2A) with the following properties: A biotin moiety (Biocytin, for avidin affinity purification) at the N-terminus followed by the FLAG-antibody epitope (DYKDDDDK, SEQ ID NO: 1), which is covalently linked to propargylglycine harboring the reactive alkyne group. A short spacer (GGA) was introduced between Biocytin and the FLAG epitope to ensure the steric accessibility of both biotin and FLAG during affinity purification. This tag can be also proteolytically cleaved by trypsin; following cleavage the resulting mass gain of tagged AHA is 107, which can be easily detected in the mass spectral analysis. Tandem purification is achieved by using first a monomeric avidin resin (Pierce) and then an anti-FLAG-M2 affinity gel (Sigma). In the first experiments, Applicants were able to demonstrate the successful use of this new tag. Fig 2B shows the subsequent purification of AHA-labeled proteins. Following tagging with the Biotin-FLAG-alkyne tag, AHA-labeled proteins from a HEK293 cell lysate were first subjected to an avidin column. After SDS elution from the matrix and removal of the majority of SDS, these proteins were further purified using an anti-FLAG-M2 affinity gel. Matrix-bound proteins were eluted by boiling in SDS sample buffer. As a control, a cell lysate from methionine-incubated HEK293 cells was treated in parallel. Note the absence of biotin signal in the methionine control. Both matrices allow for specific and mild elution of bound proteins by competition with biotin or FLAG-peptide, respectively, which will increase the specificity of the procedure even more. An establishment of these mild elution conditions is currently underway.

To assess the efficiency of the tandem purification procedure further, Applicants examine the composition of a tandem purified mixture of two proteins, GST and DHFR. One of them is expressed in *E. Coli* in the presence of AHA in minimal medium and the other is expressed in rich medium containing methionine. After [3+2]-cycloaddition with the tandem featured tag, varying ratios of the two proteins are purified together and analyzed by mass spectrometry for contamination by the non-tagged protein. Additionally, different amounts of a single, tagged protein (*e.g*., GST) are added to a non-tagged cell lysate. Tandem purification and mass spectrometry are performed to establish how much of a tagged protein is needed for successful identification. Finally, the levels of AHA labeling is varied and determined in order to optimize and characterize the method.

Further internal validation of the mass spectral data is achieved by using 10-fold deuterated L-leucine (D10-L-leucine) together with AHA during protein synthesis [35]. Peptides derived from proteins labeled with AHA + D10-L-leucine are expected to show an increase of 10 mass units per incorporated D10-L-leucine, and as such can be distinguished from peptides derived from preexisting proteins.

### References

1. Zhang et al., Chemical probes and tandem mass spectrometry: a strategy for the quantitative analysis ofproteomes and subproteomes. Curr Opin Chem Biol 8(1): 66-75, 2004.
2. Kiick et al., Incorporation of azides into recombinant proteins for chemoselective modification by the Staudinger ligation. Proc Natl Acad Sci USA 99(1): 19-24,2002.
3. Link and Tirrell, Cell Surface Labeling of Escherichia coli via Copper(I)-Catalyzed [3+1] Cycloaddition. Journal of the American Chemical Society 125(37): 11164-11165,2003.
4. Aakalu et al., Dynamic visualization of local protein synthesis in hippocampal neurons. Neuron 30(2): 489-502, 2001.
5. Huber et al., Role for rapid dendritec protein synthesis in hippocampal mGluR-dependent long-term depression. Science 288(5469): 1254-7,2000.
6. Kang and Schuman, A requirement for local protein synthesis in neurotrophin-induced hippocampal synaptic plasticity. Science 273(5280): 1402-6,1996.
7. Kacharmina et al., Stimulation of glutamate receptor protein synthesis and membrane insertion within isolated neuronal dendrites. Proc Nail Aca Sci U S A 97(21): 11545-50, 2000.
8. Ouyang et al., Visualization of the distribution of autophosphorylated calcium / calmodulin-dependent protein kinase II after tetanic stimulation in the CAI area of the hippocampas. J Neurosci 17(14): 541-627, 1997.
9. Ouyang et al., Tetanic stimulation leads to increased accumulation of Ca2+ / calmodulin-dependent protein kinase H via dendritic protein synthesis in hippocampal neurons. J Neurosci. 19(18):7823-33, 1999.
10. Steward and Levy, Preferential localization of polyribosomes under the base of dendritic spines in granule cells of the dentate gyrus. J Neurosci 2(3): 284-91,1982.
11. Steward and Fass, Polyribosomes associated with dendritic spines in the denervated dentate gyrus: evidence for local regulation of protein synthesis during reinnervation. Prog Brain Res 58: 131-6, 1983.
12. Steward and Falk, Protein-synthetic machinery at postsynaptic sites during synaptogenesis: a quantitative study of the association between polyribosomes and developing synapses. J Neurosci 6(2):412-23, 1986.
13. Nguyen et al., Requirement of a critical period of transcription for induction of a late phase of LTP. Science 265(5175): 1104-7, 1994.
14. Martin et al., Synapse-specific, long-term facilitation of aplysia sensory to motor synapses: a function for local protein synthesis in memory storage. Cell 91(7): 927-38, 1997.
15. Kang and Schuman, A requirement for local protein synthesis in neurotrophin-induced hippocampal synaptic plasticity. Science 273(5280): 1402-6,1996.
16. Casadio et al., A transient, neuron-wide form of CREB-mediated long-term facilitation can be stabilized at specific synapses by local protein synthesis. Cell 99(2): 221-37, 1999.
17. Bito et al., CREB phosphorylation and dephosphorylation: a Ca2+- and stimulus duration-dependent switch for hippocampal gene expression. Cell 87(7): 1203-14, 1996.
18. Steward and Schuman, Protein synthesis at synaptic sites on dendrites. Annu Rev Neurosci 24: 299-325, 2001.
19. Steward and Schuman, Compartmentalized synthesis and degradation of proteins in neurons. Neuron 40(2): 347-59, 2003.
20. Wu et al., CPEB-mediated cytoplasmic polyadenylation and the regulation of experience-dependent translation of alpha-CaMKII mRNA at synapses. Neuron 21(5): 1129-39, 1998.
21. Weiler et al., Synapse-activated protein synthesis as a possible mechanism of plastic neural change. Prog Brain Res 100: 189-94, 1994.
22. Steward, mRNA localization in neurons: a multipurpose mechanism? Neuron 18(1): 9-12,1997.
23. Torre and Steward, Demonstration of local protein synthesis within dendrites using a new cell culture system that permits the isolation of living axons and dendrites from their cell bodies. J Neurosci 12(3): 762-72, 1992.
24. Glanzer and Eberwine, Mechanisms of translational control in dendrites. Neurobiol Aging 24(8): 1105-11, 2003.
25. Crino and Eberwine, Molecular characterization of the dendritic growth cone: regulated mRNA transport and local protein synthesis. Neuron 17(6): 1173-87,1996.
26. Crino et al., Presence and phosphorylation of transcription factors in developing dendrites. Proc Natl Acad Sci U S A 95(5): 2313-8, 1998.
27. Job and Eberwine, Identification of sites for exponential translation in living dendrites. Proc Natl Acad Sci U S A 98(23): 13037-42, 2001.
28. Ju et al., Activity-dependent regulation of dendritic synthesis and trafficking of AMPA receptors. Nat Neurosci 7(3): 244-53, 2004.
29. Brendel et al., Characterization of Staufen 1 ribonucleoprotein complexes. Biochem J 384(Pt 2): 239-46, 2004.
30. Eberwine et al., Local translation of classes of mRNAs that are targeted to neuronal dendrites. Proc Natl Acad Sci USA 98(13): 7080-5, 2001.
31. Gygi et al., Quantitative analysis of complex protein mixtures using isotope-coded affinity tags. Nat Biotechnol 17(10): 994-9, 1999.
32. Washburn et al., Large-scale analysis of the yeast proteome by multidimensional protein identification technology. Nat Biotechnol 19(3): 242-7,2001.
33. Tabb et al., DTASelect and Contrast: tools for assembling and comparing protein identifications from shotgun proteomics. J Proteome Res 1(1): p. 21-6, 2002.
34. Ducret et al., High throughput protein characterization by automated reverse-phase chromatography / electrospray tandem mass spectrometry. Protein Sci 7(3): 706-19,1998.
35. Ong et al., Stable isotope labeling by amino acids in cell culture, SILAC, as a simple and accurate approach to expression proteomics. Mol Cell Proteomics 1(5): 376-86, 2002.
36. Bassell et al., Sorting of beta-actin mRNA and protein to neurites and growth cones in culture. J Neurosci 18(1): 251-65, 1998.
37. Kiebler et al., Purification and characterization of rat hippocampal CA3-dendritec spines associated with mossy fiber terminals. FEBS Lett 445(1): 80-6,1999.
38. Meffert et al., Nitric oxide stimulates Ca2+-independent synaptic vesicle release. Neuron 12(6): 1235-44, 1994.
39. Torre and Steward, Protein synthesis within dendrites: glycosylation of newly synthesized proteins in dendrites of hippocampal neurons in culture. J Neurosci 16(19): 5967-78, 1996.
40. Gerber et al., Absolute quantification of proteins and phosphoproteins from cell lysates by tandem MS. Proc Natl Acad Sci USA 100(12): 6940-5, 2003.
41. Peng et al., Semi-quantitative proteomic analysis of rat forebrain postsynaptic density fractions by mass spectrometry. J Biol Chem 279(20): 21003-11,2004.
42. Yin et al., The brain-derived neurotrophic factor enhances synthesis of Arc in synaptoneurosomes. Proc Natl Acad Sci U S A 99(4): 2368-73, 2002.
43. Scbratt et al., BDNF regulates the translation of a select group of mRNAs by a mammalian target of rapamycine-phosphatidylinositol 3-kinase-dependent pathway during neuronal development. J Neurosci 24(33): 7366-77, 2004.
44. Bagni et al., Chemical stimulation of synaptosomes modulates alpha - Ca2+/calmodulin-dependent protein kinase II mRNA association to polysomes. J Neurosci 20(10): RC76, 2000.
45. Agard et al., A strain promoted [3 + 2] azide-alkyne cycloaddition for covalent modification of biomolecules in living systems. J Am Chem Soc 126(46): 15046-7, 2004.
46. Mangold et al., Azidoalanine mutagenicity in Salmonella: effect of homologation and alpha-methyl substitution. Mutat Res 216(1): 27-33, 1989.
47. Wang et al., Bioconjugation by copper-(I) -catalyzed azide-alkyne [3 + 2] cycloaddition. J Am Chem Soc 125 (11): 3192-3,2003.
48. Malgaroli and Tsien, Glutamate-induced long-term potentiation of the frequency of miniature synaptic currents in cultured hippocampal neurons. Nature 357(6374): 134-9, 1992.
49. Graumann et al., Applicability of tandem affinity purification MudPIT to pathway proteomics in yeast. Mol Cell Proteomics 3(3): 226-37, 2004.

## Claims

1. A functional reagent comprising:
(1) a reactive group for reacting with a non-natural amino acid incorporated into a protein;
(2) an affinity moiety or a fluorescent moiety; and,
(3) one or more cleavable groups situated between the reactive group and the
affinity moiety or the fluorescent moiety;
wherein the reactive group is one of the following:
a) an azido group;
b) a ketone or an aldehyde moiety; or
c) a terminal alkyne moiety;
and wherein the one or more cleavable groups are selected from: photo-cleavable groups;
groups which have a recognition sequence for a protease; groups which have a recognition sequence for an endonuclease; and chemically cleavable groups selected from diols, disulfides, diazo groups, esters, sulfones, and groups comprising methionine.

2. The functional reagent of any preceding claim, which is sufficiently membrane permeable to diffuse into a live cell.

3. The functional reagent of any preceding claim, which is substantially non-toxic.

4. The functional reagent of any preceding claim, wherein the reactive group reacts with a non-natural amino acid incorporated in a protein.

5. The functional reagent of any preceding claim, further comprising an antigenic moiety that can be recognized by an antibody.

6. The functional reagent of claim 5, wherein the antigenic moiety is an epitope tag.

7. The functional reagent of claim 6, wherein the epitope tag is a FLAG tag, optionally wherein the FLAG tag comprises one or more cleavage sites for a sequence-specific protease; and further optionally wherein the sequence-specific protease is trypsin.

8. The functional reagent of claim 7, wherein the FLAG tag is situated between the affinity moiety and the reactive group.

9. The functional reagent of any preceding claim, wherein the reactive group and the affinity moiety are linked by one or more cleavable functional groups.

10. The functional reagent of any preceding claim, wherein the affinity moiety is biotin.

11. The functional reagent of any of claims 7 to 10, wherein the affinity moiety is biotin, wherein the FLAG tag comprises two cleavage sites for a sequence-specific protease, and wherein the FLAG tag is situated between the affinity moiety and the reactive group.

12. The functional reagent of any preceding claim, wherein the linker is a photo-cleavable linker.

13. The in vitro use of a functional reagent to react with a non-natural amino acid incorporated into a protein by a mammalian cell, wherein the functional reagent comprises:
(1) a reactive group to react with the non-natural amino acid; and
(2) an affinity moiety or a fluorescent moiety;
wherein the reactive group is one of the following:
a) an azido group;
b) a ketone or an aldehyde moiety; or
c) a terminal alkyne moiety.

14. The use of claim 13, wherein the functional agent further comprises one or more cleavable groups situated between the reactive group and the affinity moiety or the fluorescent moiety.

15. The use of claim 14, wherein the one or more cleavable groups are selected from:
photo-cleavable groups; groups which have a recognition sequence for a protease;
groups which have a recognition sequence for an endonuclease; and chemically cleavable groups selected from diols, disulfides, diazo groups, esters, sulfones, and groups comprising methionine.

## Patentansprüche

1. Funktionelle Reagens, die folgendes umfasst:
(1) eine reaktionsfähige Gruppe für eine Reaktion mit einer nicht-natürlichen Aminosäure, die in einem Protein inkorporiert ist;
(2) einen Affinitätsanteil oder einen fluoreszierenden Anteil; und
(3) eine oder mehrere abspaltbare Gruppen, die sich zwischen der reaktionsfähigen Gruppe und dem Affinitätsanteil oder dem fluoreszierenden Anteil befinden;
wobei es sich bei der reaktionsfähigen Gruppe um eines der folgenden handelt:
a) eine Azidogruppe;
b) ein Keton oder einen Aldehydanteil; oder
c) einen endständigen Alkynanteil;
und wobei die eine oder die mehreren abspaltbaren Gruppen ausgewählt werden aus:
photoabspaltbaren Gruppen; Gruppen mit einer Erkennungssequenz für eine Protease;
Gruppen mit einer Erkennungssequenz für eine Endonuklease; und chemisch abspaltbaren Gruppen, die ausgewählt werden aus: Diolen, Diazogruppen, Estern, Sulfonen und Gruppen,
die Methionin umfassen.

2. Funktionelle Reagens nach einem der vorstehenden Ansprüche, die ausreichend membrandurchlässig ist, um in eine Frischzelle zu diffundieren.

3. Funktionelle Reagens nach einem der vorstehenden Ansprüche, wobei diese im Wesentlichen nicht toxisch ist.

4. Funktionelle Reagens nach einem der vorstehenden Ansprüche, wobei eine reaktionsfähige Gruppe mit einer in einem Protein inkorporierten nicht-natürlichen Aminosäure reagiert.

5. Funktionelle Reagens nach einem der vorstehenden Ansprüche, wobei diese ferner einen Antigenanteil umfasst, der von einem Antikörper erkannt werden kann.

6. Funktionelle Reagens nach Anspruch 5, wobei es sich bei dem Antigenanteil um eine Epitop-Markierung handelt.

7. Funktionelle Reagens nach Anspruch 6, wobei es sich bei der Epitop-Markierung um eine FLAG-Markierung handelt, wobei die FLAG-Markierung optional eine oder mehrere Abspaltungsstellen für sequenzspezifische Protease umfasst; und wobei es sich bei der sequenzspezifischen Protease ferner optional um Trypsin handelt.

8. Funktionelle Reagens nach Anspruch 7, wobei sich die FLAG-Markierung zwischen dem Affinitätsanteil und der reaktionsfähigen Gruppe befindet.

9. Funktionelle Reagens nach einem der vorstehenden Ansprüche, wobei die reaktionsfähige Gruppe und der Affinitätsanteil durch eine oder mehrere abspaltbare funktionelle Gruppen verknüpft sind.

10. Funktionelle Reagens nach einem der vorstehenden Ansprüche, wobei es sich bei dem Affinitätsanteil um Biotin handelt.

11. Funktionelle Reagens nach einem der Ansprüche 7 bis 10, wobei es sich bei dem Affinitätsanteil um Biotin handelt, wobei die FLAG-Markierung zwei Abspaltungsstellen für eine sequenzspezifische Protease umfasst, und wobei sich die FLAG-Markierung zwischen dem Affinitätsanteil und der reaktionsfähigen Gruppe befindet.

12. Funktionelle Reagens nach einem der vorstehenden Ansprüche, wobei es sich bei dem Linker um einen photoabspaltbaren Linker handelt.

13. In vitro-Einsatz einer funktionellen Reagens für eine Reaktion mit einer nicht-natürlichen Aminosäure, die durch eine Säugetierzelle in einem Protein inkorporiert ist, wobei die funktionelle Reagens folgendes umfasst:
(1) eine reaktionsfähige Gruppe für eine Reaktion mit der nicht-natürlichen Aminosäure; und
(2) einen Affinitätsanteil oder einen fluoreszierenden Anteil;
wobei es sich bei der reaktionsfähigen Gruppe um eines der folgenden handelt:
a) eine Azidogruppe;
b) ein Keton oder einen Aldehydanteil; oder
c) einen endständigen Alkynanteil;

14. Einsatz nach Anspruch 13, wobei die funktionelle Substanz ferner eine oder mehrere abspaltbare Gruppen umfasst, die sich zwischen der reaktionsfähigen Gruppe und dem Affinitätsanteil oder dem fluoreszierenden Anteil befinden.

15. Einsatz nach Anspruch 4, wobei die eine oder die mehreren abspaltbaren Gruppen ausgewählt werden aus: photoabspaltbaren Gruppen; Gruppen mit einer Erkennungssequenz für eine Protease; Gruppen mit einer Erkennungssequenz für eine Endonuklease; und chemisch abspaltbaren Gruppen, die ausgewählt werden aus: Diolen, Diazogruppen, Estern, Sulfonen und Gruppen, die Methionin umfassen.

## Revendications

1. Réactif fonctionnel comprenant :
(1) un groupe réactif pour réagir avec un acide aminé non naturel incorporé dans une protéine ;
(2) une fraction d'affinité ou une fraction fluorescente ; et,
(3) un ou plusieurs groupes clivables situés entre le groupe réactif et la fraction d'affinité ou la fraction fluorescente ;
dans lequel le groupe réactif est l'un des suivants :
a) un groupe azido ;
b) une fraction cétone ou aldéhyde ; ou
c) une fraction alcyne terminale ;
et dans lequel le ou les plusieurs groupes clivables sont choisis parmi : groupes photo-clivables ; groupes qui ont une séquence de reconnaissance pour une protéase ; groupes qui ont une séquence de reconnaissance pour une endonucléase ; et groupes chimiquement clivables choisis parmi les diols, disulfures, groupes diazo, esters, sulfones, et groupes comprenant de la méthionine.

2. Réactif fonctionnel selon l'une quelconque des revendications précédentes, qui est à membrane suffisamment perméable pour se diffuser dans une cellule vivante.

3. Réactif fonctionnel selon l'une quelconque des revendications précédentes, qui est sensiblement non toxique.

4. Réactif fonctionnel selon l'une quelconque des revendications précédentes, dans lequel le groupe réactif réagit avec un acide aminé non naturel incorporé dans une protéine.

5. Réactif fonctionnel selon l'une quelconque des revendications précédentes, comprenant en outre une fraction antigénique qui peut être reconnue par un anticorps.

6. Réactif fonctionnel selon la revendication 5, dans lequel la fraction antigénique est un marqueur d'épitope.

7. Réactif fonctionnel selon la revendication 6, dans lequel le marqueur d'épitope est un marqueur FLAG, éventuellement dans lequel le marqueur FLAG comprend un ou plusieurs sites de clivage pour une protéase spécifique de la séquence ; et en outre éventuellement dans lequel la protéase spécifique de la séquence est la trypsine.

8. Réactif fonctionnel selon la revendication 7, dans lequel le marqueur FLAG est situé entre la fraction d'affinité et le groupe réactif.

9. Réactif fonctionnel selon l'une quelconque des revendications précédentes, dans lequel le groupe réactif et la fraction d'affinité sont liés par un ou plusieurs groupes fonctionnels clivables.

10. Réactif fonctionnel selon l'une quelconque des revendications précédentes, dans lequel la fraction d'affinité est la biotine.

11. Réactif fonctionnel de l'une quelconque des revendications 7 à 10, dans lequel la fraction d'affinité est la biotine, dans lequel le marqueur FLAG comprend deux sites de clivage pour une protéase spécifique de la séquence, et dans lequel le marqueur FLAG est situé entre la fraction d'affinité et le groupe réactif.

12. Réactif fonctionnel selon l'une quelconque des revendications précédentes, dans lequel le lieur est un lieur photo-clivable.

13. Utilisation in vitro d'un réactif fonctionnel pour réagir avec un acide aminé non naturel incorporé dans une protéine par une cellule de mammifère, dans lequel le réactif fonctionnel comprend :
(1) un groupe réactif pour réagir avec l'acide aminé non naturel ; et
(2) une fraction d'affinité ou une fraction fluorescente ;
dans lequel le groupe réactif est l'un des suivants :
a) un groupe azido ;
b) une fraction cétone ou aldéhyde ; ou
c) une fraction alcyne terminale.

14. Utilisation selon la revendication 13, dans laquelle l'agent fonctionnel comprend en outre un ou plusieurs groupes clivables situés entre le groupe réactif et la fraction d'affinité ou la fraction fluorescente.

15. Utilisation selon la revendication 14, dans laquelle le ou les plusieurs groupes clivables sont choisis parmi : groupes photo-clivables ; groupes qui ont une séquence de reconnaissance pour une protéase ; groupes qui ont une séquence de reconnaissance pour une endonucléase ; et groupes chimiquement clivables choisis parmi les diols, disulfures, groupes diazo, esters, sulfones, et groupes comprenant de la méthionine.
